# EUROPEAN PATENT SPECIFICATION

(11) **EP 4 415 630 B1**
(45) Date of publication and mention of the grant of the patent: **09.12.2026**
(21) Application number: 22792908.0
(22) Date of filing: 05.10.2022
(51) Int. Cl.: A61B 17/072

(54) **SURGICAL STAPLING DEVICE WITH FIRING LOCKOUT MECHANISM**
CHIRURGISCHE KLAMMERVORRICHTUNG MIT AUSLÖSESPERRMECHANISMUS
DISPOSITIF D'AGRAFAGE CHIRURGICAL COMPORTANT UN MÉCANISME DE VERROUILLAGE DE DÉCLENCHEMENT

(30) Priority: 13.10.2021 US 202117500051
(43) Date of publication of application: 21.08.2024
(73) Proprietor: Covidien LP, Mansfield, MA 02048 (US)
(72) Inventor: FERNANDES, Roanit, North Haven, CT 06473 (US); CAPPOLA, Kenneth M., North Haven, CT 06473 (US)
(74) Representative: Zimmermann & Partner Patentanwälte mbB
(86) International application number: PCT/IB2022/059499
(87) International publication number: WO 2023/062482

(56) References cited:
- EP-A1- 3 420 938
- CN-A- 110 099 618
- US-A1- 2019 125 342
- US-A1- 2019 167 266

## Description

### FIELD

The disclosure is directed to surgical stapling devices having staple cartridges and, more particularly, to a surgical stapling device with a lockout mechanism to prevent firing of the stapling device when a knife is not present in the staple cartridge.

### BACKGROUND

Surgical stapling devices for simultaneously stapling and cutting tissue are well known in the art and include a tool assembly and a drive assembly. The tool assembly includes an anvil assembly and a cartridge assembly having a channel member and a staple cartridge that is received within the channel member. Typically, the staple cartridge includes a cartridge body that supports staples, pushers, and an actuation sled. The actuation sled is movable through the cartridge body from a retracted position to an advanced position into sequential engagement with the pushers to sequentially eject the staples from the cartridge body. In some stapling devices, a knife is supported on the actuation sled and is movable with the actuation sled through the cartridge body. The drive assembly is positioned proximally of the actuation sled and is movable in relation to the anvil and cartridge assemblies to advance the actuation sled and knife through the staple cartridge to move the tool assembly between open and clamped positions, to eject the staples from the cartridge body, and to cut tissue clamped between the anvil and cartridge assemblies.

When a stapling device is fired when a knife is not present in the staple cartridge, the staples will be ejected through the tissue but the tissue will not be cut. A continuing need exists for a lock mechanism of simple construction that can prevent advancement of a drive assembly of a stapling device when a knife is not present in the staple cartridge.

EP 3420938 A1 discloses a surgical instrument, comprising an articulatable end effector that is selectively articulatable about an articulation axis. The end effector comprises first and second jaws wherein the second jaw is pivotable relative to the first jaw about a jaw pivot axis. The instrument includes an axially movably firing member that includes at least one jaw engagement feature that is configured to apply a closure motion to the second end effector jaw as the axially movable firing member is moved from a starting position to an end position within the first end effector jaw. Each jaw engagement feature is configured such that a portion thereof is positioned between the jaw pivot axis and the articulation axis when the axially movable firing member is in the starting position. Further, the disclosed surgical tool assembly employs a firing member lockout system.

US 2019/125342 A1 discloses a staple cartridge assembly which has a sled that carries a knife rotatably supported on the sled.

US 2019/167266 A1 discloses drive assemblies for surgical clamping and cutting instruments, as well as surgical clamping and cutting instruments using these drive assemblies, are disclosed. The drive assemblies may include a drive member, a locking member mounted to the drive member, and a spring. The drive member may be configured to releasably engage at least one of a knife or a shuttle of a surgical clamping and cutting instrument for translating the knife and/or shuttle in a distal direction through a firing stroke. The locking member may be movable from a first position permitting distal translation of the drive member through the firing stroke, and a second position inhibiting distal translation of the drive member through the firing stroke. The spring may be configured to bias the locking member toward the second position.

### SUMMARY

The present invention is defined by the features of the independent claim. Further embodiments are provided by the dependent claims.

This disclosure is directed to a surgical stapling device that includes a tool assembly and a drive assembly that supports a lockout mechanism. The lockout mechanism is configured to interact with the tool assembly when a knife is not present within the tool assembly to prevent firing of the stapling device.

Aspects of this disclosure are directed to a surgical stapling device including an elongate body, a tool assembly, and a drive assembly. The elongate body has a proximal portion and a distal portion. The tool assembly is supported on the distal portion of the elongate body and includes a cartridge assembly and an anvil assembly that are coupled together such that the tool assembly is movable between open and clamped positions. The tool assembly defines a notch. The cartridge assembly includes a staple cartridge that has a cartridge body, staples, pushers, and an actuation sled and knife assembly. The actuation sled and knife assembly includes an actuation sled and a knife supported on the actuation sled for movement between raised and lowered positions. The actuation sled and knife assembly is movable within the cartridge body from a retracted position to an advanced position to eject the staples from the cartridge body. The drive assembly is movable between a retracted position and an advanced position and includes a flexible drive beam, a working member, and a lockout mechanism. The flexible drive beam has a proximal portion and a distal portion. The working member is supported on the distal portion of the flexible drive beam and includes a first beam, a second beam, and a vertical strut that connects the first beam to the second beam. The lockout mechanism is supported on the working member and is movable between a locked position and an unlocked position. In the locked position, the lockout mechanism is received within the notch defined by the tool assembly to prevent movement of the drive assembly to the advanced position.

Other aspects of the disclosure are directed to a drive assembly that includes a flexible drive beam, a working member, and a lockout mechanism. The flexible drive beam has a proximal portion and a distal portion. The working member is supported on the distal portion of the flexible drive beam and includes a first beam, a second beam, and a vertical strut that connects the first beam to the second beam. The lockout mechanism is supported on the working member and is movable between a locked position and an unlocked position.

Aspects of the disclosure are also directed to a tool assembly including a cartridge assembly an anvil assembly, and a drive assembly. The cartridge assembly includes a staple cartridge that has a cartridge body, staples, pushers, and an actuation sled and knife assembly. The actuation sled and knife assembly includes an actuation sled and a knife that is supported on the actuation sled for movement between raised and lowered positions. The actuation sled and knife assembly is movable within the cartridge body from a retracted position to an advanced position to eject the staples from the cartridge body. The anvil assembly is coupled to the cartridge assembly such that the tool assembly is movable between open and clamped positions. The drive assembly is movable between a retracted position and an advanced position and includes a flexible drive beam, a working member, and a lockout mechanism. The flexible drive beam has a proximal portion and a distal portion and the working member is supported on the distal portion of the flexible drive beam. The working member includes a first beam, a second beam, and a vertical strut that connects the first beam to the second beam. The lockout mechanism is supported on the working member and is movable between a locked position and an unlocked position. In the locked position, the lockout mechanism is received within the notch defined by the tool assembly to prevent movement of the drive assembly to the advanced position.

The lockout mechanism includes a lockout member that is pivotably supported on the working member of the drive assembly.

The vertical strut of the working member defines a cutout and the lockout member is confined within the cutout in the unlocked position and extends from the cutout in the locked position.

In certain aspects of the disclosure, the lockout mechanism includes a biasing member that is positioned to urge the lockout member towards the locked position.

In aspects of the disclosure, the vertical strut defines a cam channel that extends upwardly in a proximal direction along the vertical strut.

In some aspects of the disclosure, the cam channel communicates with the cutout.

In certain aspects of the disclosure, the lockout member includes a finger that extends into the cam channel.

In aspects of the disclosure, the knife includes a protrusion that is received in the cam channel when the drive assembly is moved from its retracted position towards its advanced position.

In some aspects of the disclosure, the protrusion is movable through the cam channel to move the knife from the lowered position to the raised position.

In certain aspects of the disclosure, the protrusion of the knife is movable into engagement with the finger of the lockout member to move the lockout member from the locked position to the unlocked position.

In aspects of the disclosure, the notch is defined in the anvil assembly.

In some aspects of the disclosure, the anvil assembly defines a knife slot and the notch is formed along the knife slot.

In certain aspects of the disclosure, the stapling device includes a handle assembly that is coupled to the proximal portion of the elongate body.

In aspects of the disclosure, the actuation sled includes a body having a central portion and cam surfaces positioned on opposite sides of the central portion.

Other features of the disclosure will be appreciated from the following description.

### BRIEF DESCRIPTION OF THE DRAWINGS

Various aspects of the disclosure are described herein below with reference to the drawings, wherein:
FIG. 1 is side perspective view of a stapling device according to aspects of the disclosure including a tool assembly in an open position;
FIG. 2 is a side perspective view of the tool assembly of the stapling device shown in FIG. 1 with a staple cartridge separated from a channel member of a cartridge assembly of the stapling device;
FIG. 3 is an exploded view of the staple cartridge of the cartridge assembly shown in FIG. 2;
FIG. 4 is an enlarged view of the indicated area of detail shown in FIG. 2 illustrating the proximal portion of the staple cartridge;
FIG. 5 is a side perspective view of an actuation sled and knife assembly of the staple cartridge shown in FIG. 2;
FIG. 5A is an exploded side perspective view of the actuation sled and knife assembly shown in FIG. 5;
FIG. 6 is a bottom perspective view of an anvil assembly of the tool assembly shown in FIG. 2;
FIG. 7 is an exploded view of the anvil assembly shown in FIG. 6;
FIG. 8 is an enlarged view of the indicated area of detail shown in FIG. 2 illustrating one side of a distal portion of a drive assembly of the stapling device shown in FIG. 1;
FIG. 9 is a perspective view from an opposite side of the distal portion of the drive assembly shown in FIG. 8;
FIG. 10 is side perspective view of the distal portion of the drive assembly shown in FIG. 9 with a lockout mechanism separated from the drive assembly;
FIG. 11 is a side perspective view of a lockout member of the lockout mechanism shown in FIG. 10;
FIG. 12 is a side perspective view of a proximal portion of the tool assembly of the stapling device shown in FIG. 1 as the drive assembly is advanced towards the actuation sled to move the tool assembly to the clamped position and to move the lockout mechanism to an unlocked position;
FIG. 13 is a side perspective view from one side of the actuation sled and knife assembly and the distal portion of the drive assembly in the clamped and pre-fired position;
FIG. 14 is a side perspective view from an opposite side of the actuation sled and knife assembly and the distal portion of the drive assembly with the lockout mechanism removed in the clamped and pre-fired position;
FIG 15 is a perspective view from a distal end of the actuation sled and knife assembly and the distal portion of the drive assembly in the clamped and pre-fired position;
FIG. 16 is a bottom perspective view of the tool assembly of the stapling device shown in FIG. 1 illustrating the anvil assembly, the actuation sled and knife assembly, and the distal portion of the drive assembly as a firing stroke begins, with the remaining components of the tool assembly shown in phantom;
FIG. 17 is an enlarged view of the indicated area of detail shown in FIG. 16;
FIG. 18 is a bottom perspective view of the tool assembly of the stapling device shown in FIG. 1 illustrating the anvil assembly, the actuation sled and knife assembly, and the distal portion of the drive assembly after the stapling device has been fired and the drive assembly has returned to its retracted position, with the remaining components of the tool assembly shown in phantom; and
FIG. 19 is an enlarged view of the indicated area of detail shown in FIG. 1.

### DETAILED DESCRIPTION

The disclosed surgical stapling device will now be described in detail with reference to the drawings in which like reference numerals designate identical or corresponding elements in each of the several views. However, it is to be understood that aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

In this description, the term "endoscopic" is used to include endoscopic, laparoscopic, arthroscopic, and any other procedure conducted through small incisions, or cannulas positioned through small incisions in the skin. In addition, the term "proximal" is used generally to refer to that portion of the device that is closer to a clinician during use of the stapling device in its customary manner, while the term "distal" is used generally to refer to that portion of the device that is farther from the clinician during use of the stapling device in its customary manner. Further, the term "clinician" is used generally to refer to medical personnel including doctors, nurses, and support personnel. Moreover, directional terms such as front, rear, upper, lower, top, bottom, and similar terms are used to assist in understanding the description and are not intended to limit the disclosure.

The disclosed surgical stapling device includes a tool assembly and a drive assembly that supports a lockout mechanism. The lockout mechanism is configured to interact with the tool assembly when a knife is not present within the tool assembly to prevent firing of the stapling device.

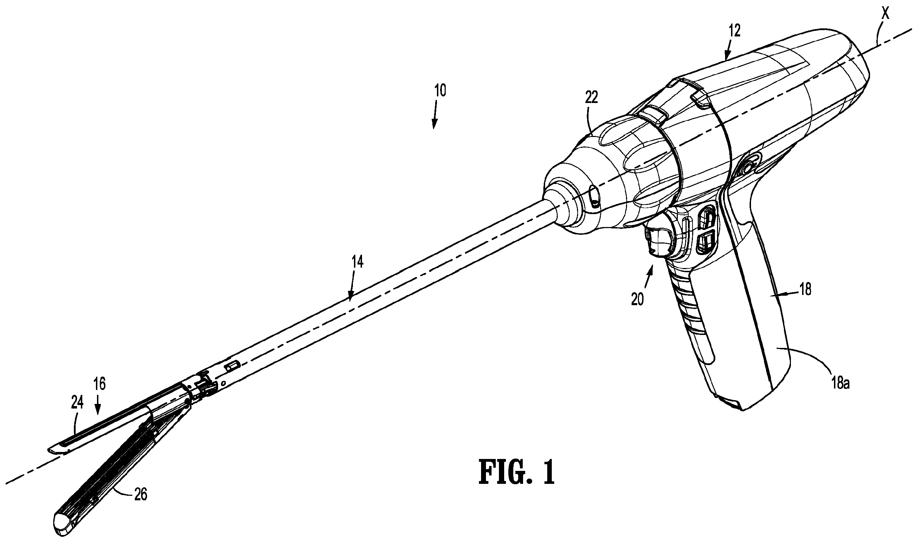

FIG. 1 illustrates a surgical stapling device according to aspects of the disclosure shown generally as stapling device 10. The stapling device 10 includes a handle assembly 12, an elongate body 14, and a tool assembly 16. The elongate body 14 defines a longitudinal axis "X" (FIG. 1). The handle assembly 12 includes a body 18 that defines a hand grip 18a, a plurality of actuator buttons 20, and a rotation knob 22. The rotation knob 22 is rotatably supported on a distal portion of the body 18 of the handle assembly 12 and supports the elongate body 14 to facilitate rotation of the elongate body 14 and the tool assembly 16 in relation to the handle assembly 12 about the longitudinal axis "X". The actuator buttons 20 control operation of the various functions of the stapling device 10 including articulation, clamping, firing, and cutting of tissue. The tool assembly 16 includes an anvil assembly 24 and a cartridge assembly 26 that are movable in relation to each other between open and clamped positions. Although the stapling device 10 is illustrated as an electrically powered stapling device, it is envisioned that the disclosed tool assembly 16 would also be suitable for use with manually powered surgical stapling devices and robotically operated stapling devices. U.S. Patent No. 9,055,943 discloses a surgical stapling device including a powered handle assembly, U.S. Patent No. 6,241,139 discloses a surgical stapling device including a manually actuated handle assembly, and U.S Patent No. 9,962,159 discloses a stapling device that is configured for use with a robotic system.

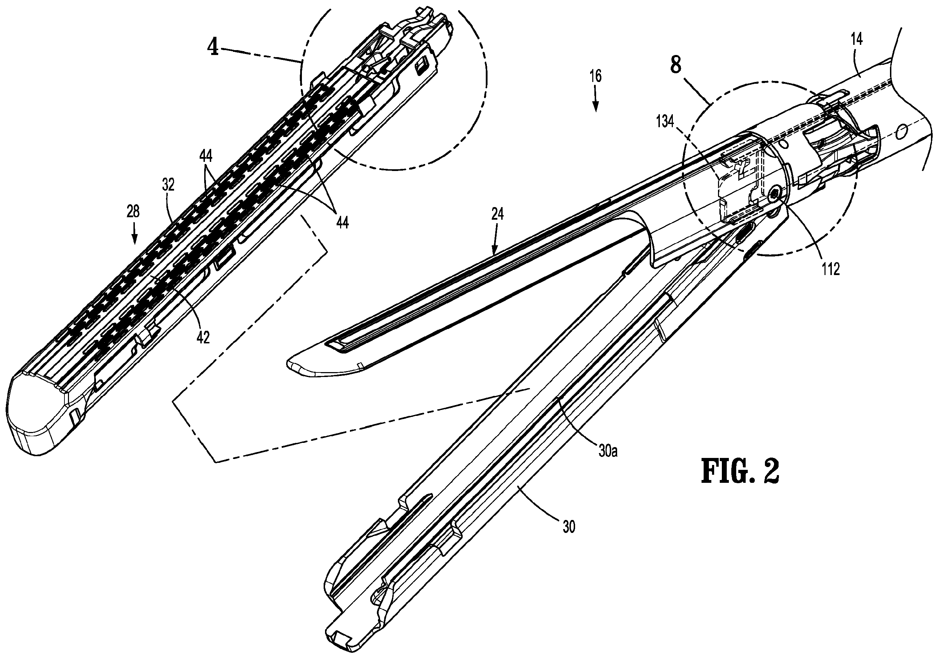

FIGS. 2-5 illustrate the cartridge assembly 26 which includes a staple cartridge 28 and a channel member 30 that defines a recess 30a that receives the staple cartridge 28. In aspects of the disclosure, the staple cartridge 28 is releasably supported within the recess 30a of the channel member 30 and is replaceable to facilitate reuse of the stapling device 10.

The staple cartridge 28 includes a cartridge body 32, an actuation sled and knife assembly 34, pushers 36, staples 38, and a base plate 40. The cartridge body 32 defines a central knife slot 42 and staple receiving pockets 44. In aspects of the disclosure, the staple receiving pockets 44 are aligned in rows that are positioned on each side of the central knife slot 42. Although three rows of staple receiving pockets 44 are shown on each side of the central knife slot 42, it is envisioned that the cartridge body 32 may define one or more rows of staple receiving pockets 44 on each side of the central knife slot 42. Each of the staple receiving pockets 44 receives one of the staples 38 and a pusher 36. The pushers 36 support the staples 38 and are engaged by an actuation sled 34a of the actuation sled and knife assembly 34 as the actuation sled and knife assembly 34 is advanced through the cartridge body 32 to eject the staples 38 from the cartridge body 32. The base plate 40 is secured to the bottom of the cartridge body 32 to prevent the pushers 36 and staples 38 from falling from the cartridge body 32.

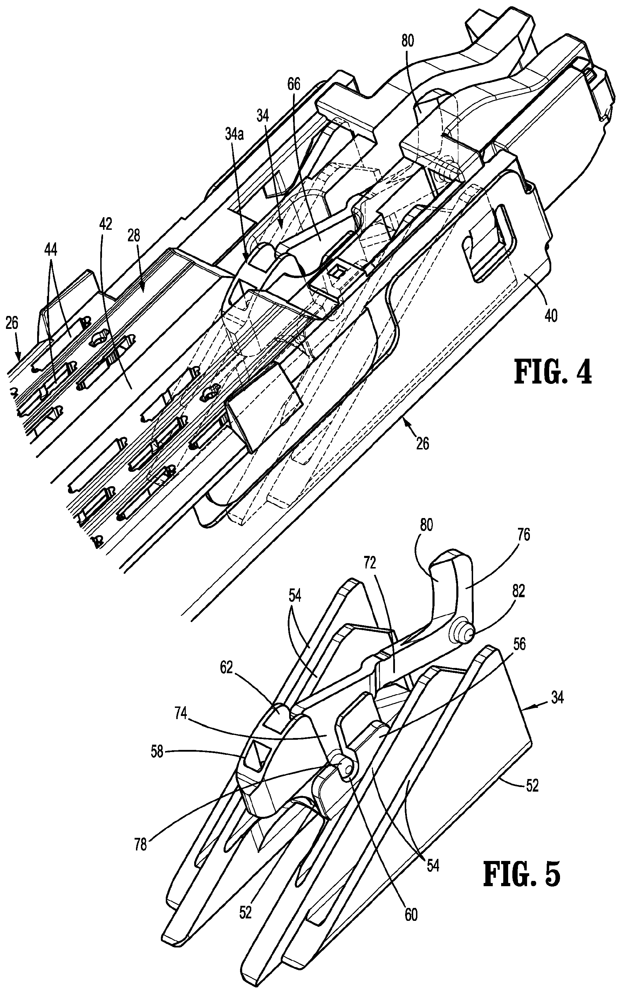

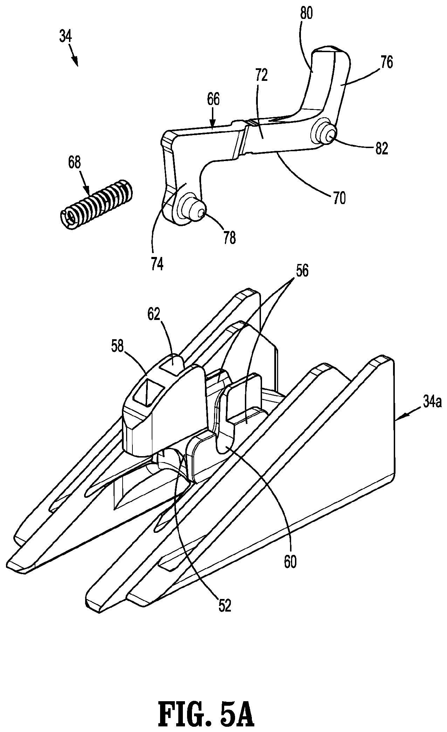

The actuation sled and knife assembly 34 includes the actuation sled 34a, a knife 66, and a biasing member 68. The actuation sled 34a supports the knife 66 and is movable within the cartridge body 32 from a retracted position to an advanced position. The actuation sled 34a includes a body 50 (FIG. 5A) that has a central portion 52 and cam surfaces 54 that are positioned on opposite sides of the central portion 52 of the body 50. The cam surfaces 54 define ramps that are positioned to engage the pushers 36 as the actuation sled 34a moves from its retracted position towards its advanced position to sequentially eject the staples 38 from the cartridge body 32.

The central portion 52 of the actuation sled 34a includes spaced walls 56 and a housing 58. Each of the spaced walls 56 defines a circular recess 60 (FIG. 5A). The housing 58 extends distally of and is positioned between longitudinal axes defined by the spaced walls 56. The housing 58 also defines a recess 62 that is aligned with the central knife slot 42 of the cartridge body 32. The central portion 52 of the actuation sled 34a supports the knife 66.

The knife 66 has a body 70 that has a Z-shaped configuration and includes an elongate arm 72, a first portion 74 that extends downwardly from a distal end of the elongate arm 72, and a second portion 76 that extends upwardly from a proximal end of the elongate arm 72. The first portion 74 of the body 70 of the knife 66 supports a pivot member 78 that is received within the circular recesses 60 in the spaced walls 56 of the actuation sled 34a such that the knife 66 is pivotably supported on the actuation sled 34a between raised and lowered positions. The actuation sled and knife assembly 34 is movable through the cartridge body 32 between retracted and advanced positions. In the raised position of the knife 66, the first portion 74 of the body 70 of the knife 66 is received within the recess 62 of the housing 58 of the central portion 52 of the actuation sled 34a. The second portion 76 of the body 70 of the knife 66 defines a distally facing cutting edge 80 that is recessed within the central knife slot 42 of the cartridge body 32 when the knife 66 is in the lowered position and extends above the central knife slot 42 when the knife 66 is in the raised position. The body 70 of the knife 66 also includes a protrusion 82 that extends outwardly of the elongate arm 72.

The actuation sled and knife assembly 34 includes a biasing member 68. The biasing member 68 is received within the recess 62 of the housing 58 of the central portion 52 of the actuation sled 34a and engages the first portion 74 of the body 70 of the knife 66 at a position above an axis defined by the pivot member 78 of the knife 66 to urge the knife 66 towards the lowered position. In aspects of the disclosure, the biasing member 68 includes a coil spring although it is envisioned that other types of biasing mechanisms or members could be used in place of the coil spring.

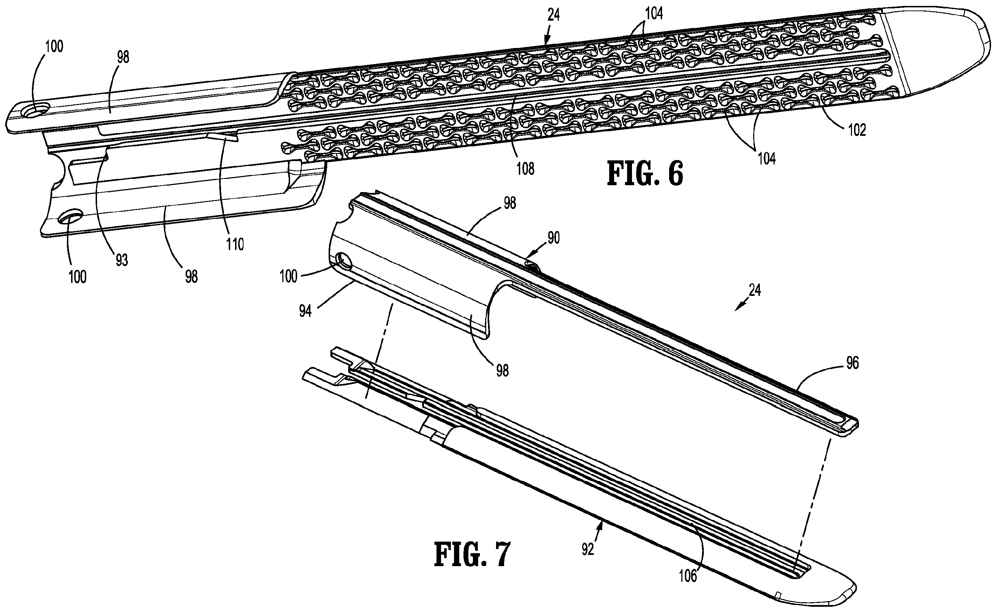

FIGS. 6 and 7 illustrate the anvil assembly 24 which includes an anvil cover 90 and an anvil plate 92. The anvil cover 90 includes a mounting portion 94, and a cover portion 96 that extends along the distal portion of the anvil plate 92. The mounting portion 94 includes spaced extensions 98 that define bores 100. The anvil plate 92 includes a tissue engaging surface 102 that is in juxtaposed relation to the staple cartridge 28 when the tool assembly 16 is in a clamped position. The tissue engaging surface 102 defines a plurality of staple deforming recesses 104. The anvil plate 92 has a side opposite to the tissue engaging surface 102 that defines an elongated recess 106. The anvil cover 90 is secured to the anvil plate 92, e.g., by welding or crimping, such that the cover portion 96 of the anvil cover 90 encloses the elongated recess 106 of the anvil plate 92 to define a channel 93 (FIG. 6) within the anvil assembly 24. The anvil plate 92 also defines a central knife slot 108 that extends through the tissue engaging surface 102 of the anvil plate 92 and communicates with the elongated recess 106 of the anvil plate 92. In aspects of the disclosure, the anvil plate 92 defines a notch 110 that is positioned proximally of the tissue engaging surface 102 of the anvil plate 102 and communicates with the central knife slot 108.

The bores 100 in the extensions 98 of the anvil cover 90 receive pivot members 112 (FIG. 2 that extend through the bores 100 into bores (not shown) defined in a proximal portion of the channel member 30 to pivotably secure the anvil assembly 24 to the cartridge assembly 26. In aspects of the disclosure, the cartridge assembly 26 can pivot towards the anvil assembly 24 between the open and clamped positions. It is envisioned that the anvil assembly 24 could pivot toward the cartridge assembly 26.

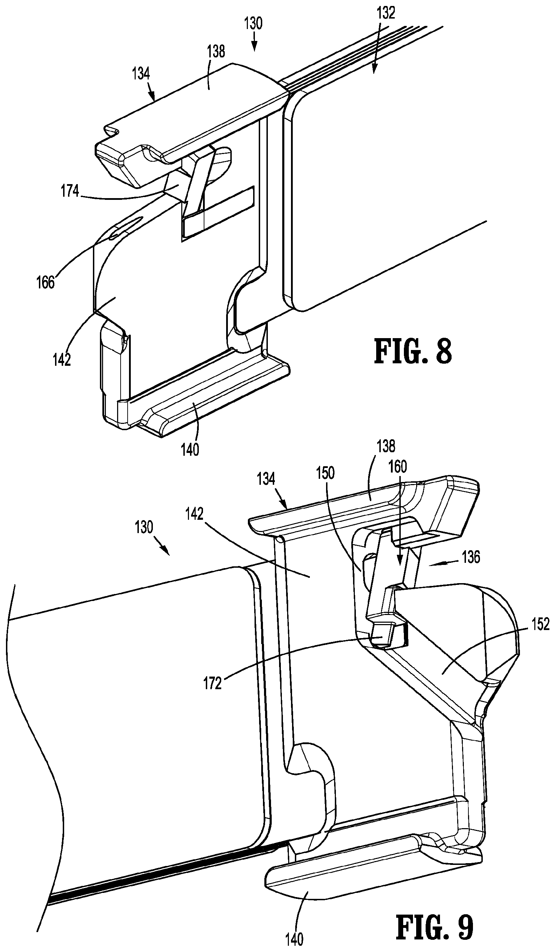

FIGS. 8-10 illustrate a drive assembly 130 of the stapling device 10 (FIG. 1) which includes a resilient and/or flexible drive beam 132, a working member 134, and a lockout mechanism 136. The resilient drive beam 132 has a proximal portion (not shown) that is coupled to a drive rod (not shown) supported within the elongate body 14 of the stapling device 10 (FIG. 1) and a distal portion that is coupled to the working member 134. In aspects of the disclosure, the resilient drive beam 132 is formed from laminated sheets of material, e.g., steel, that are welded to the working member 134. Alternately other materials of construction and securement techniques are envisioned.

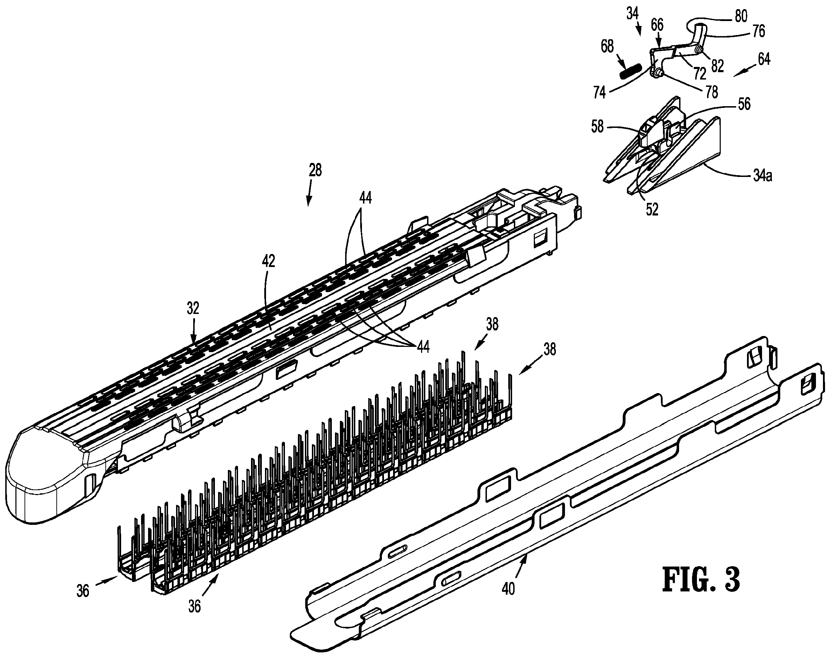

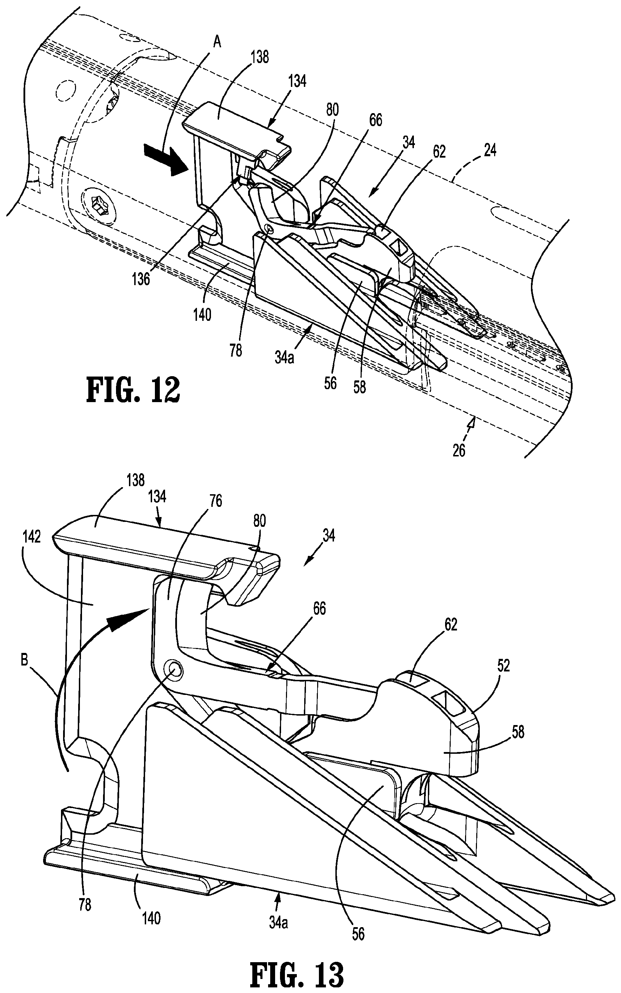

The working member 134 of the drive assembly 130 has an I-beam configuration and includes a first beam 138, a second beam 140, and a vertical strut 142 that connects the first beam 138 to the second beam 140. The working member 134 is positioned proximally of the actuation sled 34a (FIG. 12) and is movable through the tool assembly 16 between retracted and advanced positions to move the actuation sled and knife assembly 34 through the tool assembly 16 (FIG. 1) from its retracted position to its advanced position. As the working member 134 moves through the cartridge assembly 26, the first beam 138 is received within the channel 93 (FIG. 6) defined within the anvil assembly 24, the second beam 140 is engaged with the cartridge assembly 26, and the vertical strut 142 moves through the central knife slots 42 (FIG. 3) and 108 (FIG. 6) of the cartridge body 32 and the anvil plate 92, respectively.

The vertical strut 142 defines a cutout 150 and a cam channel 152. The cutout 150 extends downwardly from the first beam 138 towards the second beam 140 as shown in FIG. 9. The cam channel 152 is angled upwardly and proximally from a central portion of the vertical strut 142 and communicates with a bottom of the cutout 150. The cutout 150 receives the lockout mechanism 136.

The lockout mechanism 136 includes a lockout member 160 that is pivotally coupled to the vertical strut 142 within the cutout 150 by a pivot member 164. The pivot member 164 extends through a longitudinally extending bore 166 that extends across the cutout 150. The pivot member 164 is received within a bore 168 defined in the lockout member 160 such that the lockout member 160 can pivot laterally between an unlocked position confined within the cutout 150 and a locked position extending laterally from the cutout 150.

The lockout member 160 has a body 170 and a downwardly extending finger 172. In aspects of the disclosure, the body 170 has a substantially rectangular configuration and includes a distal stop surface 173 and a lateral protrusion 174. When the lockout member 160 is received within the cutout 150, the finger 172 is positioned in the proximal portion of the cam channel 152 and the lateral protrusion 174 is received between the first beam 138 and a surface of the vertical strut 142. In aspects of the disclosure, the finger 172 of the lockout member 160 has a tapered engagement surface 176 and increases in width in the proximal direction.

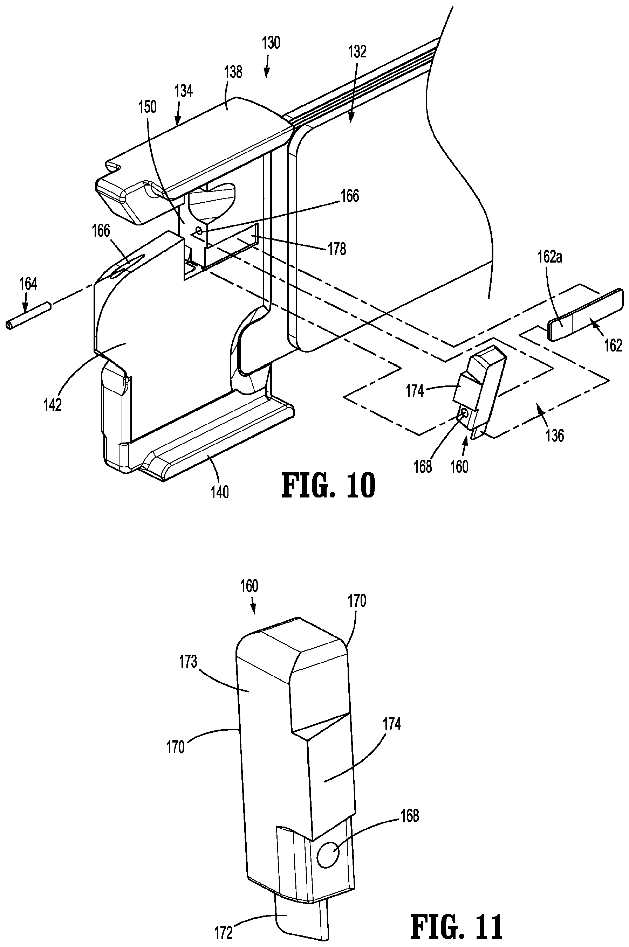

The biasing member 162 is secured within a recess 178 (FIG. 10) defined within the vertical strut 142 of the working member 134 and includes a distal portion 162a that is positioned to engage one side of the finger 172 at a position below the pivot member 164. In aspects of the disclosure, the biasing member 162 is in the form of a leaf spring that is formed of spring steel and is secured to the vertical strut by welding. Alternately, other spring types and materials are envisioned. The biasing member 162 is positioned to urge the finger 172 into the cam channel 152 to urge the portion of the lockout member 160 above the pivot member 164 from the cutout 150 to expose the stop surface 173.

When the drive assembly 130 is in its retracted position, the working member 134 is spaced proximally of the actuation sled and drive assembly 34 such that the protrusion 82 on the knife 66 is aligned with and positioned proximally of the cam channel 152 (FIG. 9) in the working member 134 of the drive assembly 130. When the drive assembly 130 is advanced from its retracted position to a clamped position in the direction of arrow "A" in FIG. 12, the working member 134 of the drive assembly 130 moves into engagement with the tool assembly 16 to move the tool assembly 16 from the open position (FIG. 1) to the clamped position. As the drive assembly 130 moves into engagement with the tool assembly 16, the vertical strut 142 of the working member 134 of the drive assembly 130 is still spaced from the central portion of the actuation sled 34a of the actuation sled and knife assembly 34.

When the drive assembly 130 begins to advance from its clamped position towards its advanced position, the protrusion 82 on the knife 66 is received in the cam channel 152 in the vertical strut 142. As the drive assembly 130 continues to advance, the protrusion 82 of the knife 66 moves through the cam channel 152 to pivot the knife 66 about the pivot member 78 in the direction of arrow "B" in FIG. 13 from the lowered position to the raised position. When the knife 66 is in the raised position, the vertical strut 142 of the working member 134 of the drive assembly 130 is positioned in abutting relation to the central portion 52 of the actuation sled 34a such that further advancement of the drive assembly 130 advances the actuation sled and knife assembly 34 through the cartridge body 32 of the staple cartridge 28.

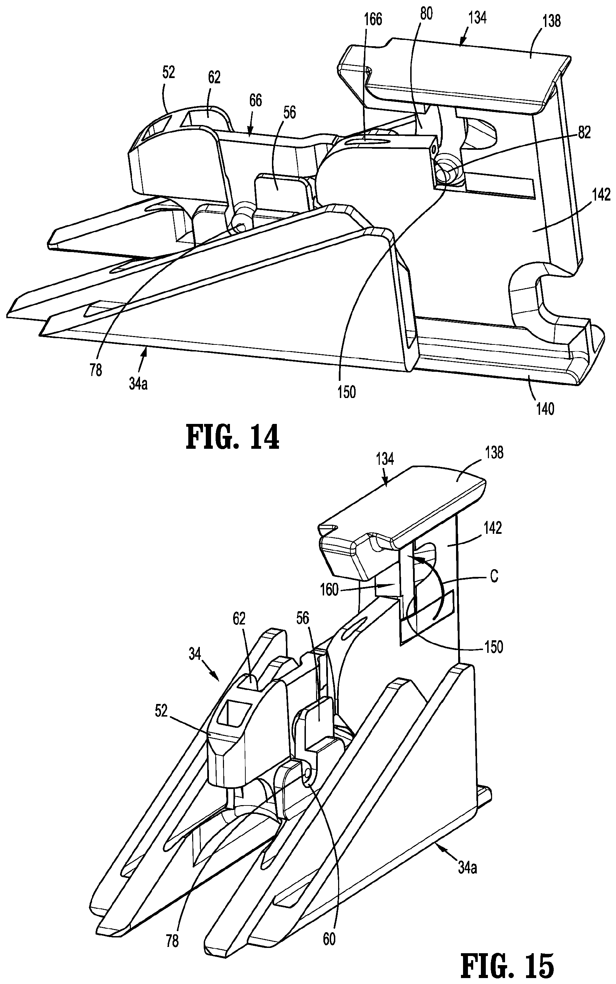

As the protrusion 82 reaches the proximal portion of the cam channel 152 (FIG. 9) as illustrated in FIGS. 14 and 15, the protrusion 82 engages the finger 172 (FIG. 9) of the lockout member 160 of the lockout mechanism 136 to pivot the lockout member 160 in the direction of arrow "C" in FIG. 15 about the pivot member 164 (FIG. 10) from the locked position to the unlocked position. In the unlocked position, the lockout member 160 is confined within the cutout 150 of the vertical strut 142 of the working member 134 and the lockout member 160 can pass by the notch 110 (FIG. 7) defined in the anvil assembly 24.

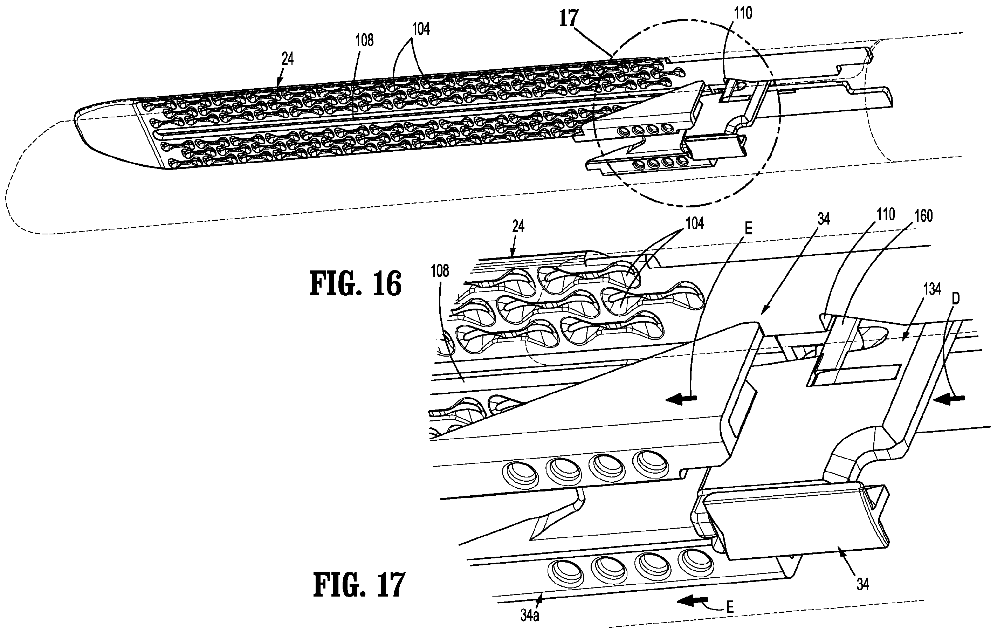

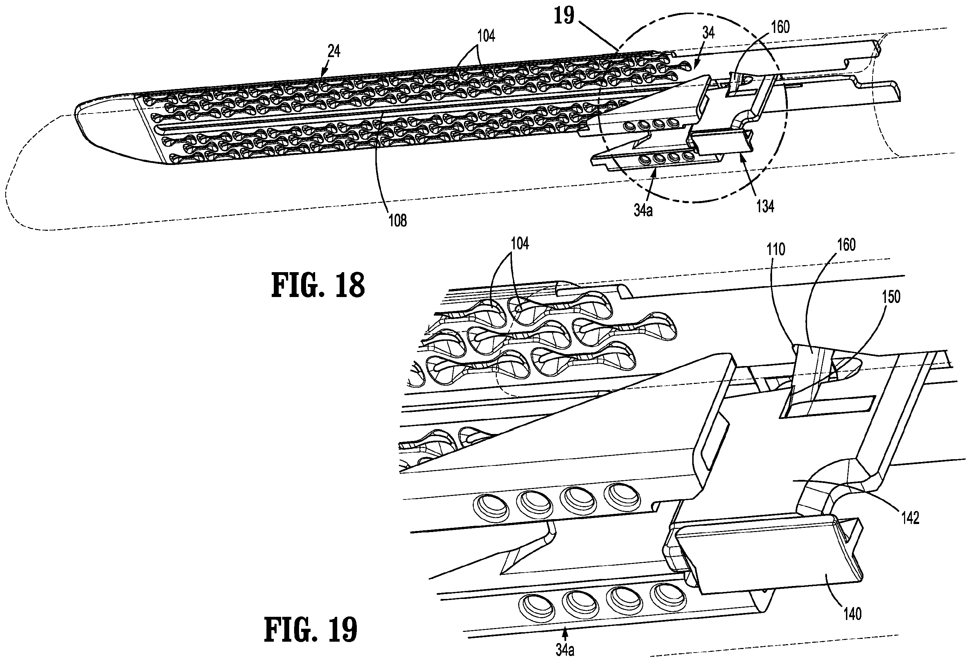

FIGS. 16-19 illustrate the actuation sled 34a, the anvil assembly 24, and the working member 134 of the drive assembly 130 when a knife (FIG. 13) is not present in the staple cartridge 28 of the cartridge assembly 26. When a knife 66 is not present in the staple cartridge 28 and the drive assembly 130 is advanced in the direction of arrow "D" in FIG. 17 to advance the actuation sled 34a in the direction of arrows "E" in FIG. 17, the lockout mechanism 136 remains in the locked position. More specifically, since no knife 66 is present in the staple cartridge 28, the lockout member 160 of the lockout mechanism 136 is not engaged by the protrusion 82 on the knife 66 and thus, remains in the locked position. In the locked position, the lockout member 160 is urged outwardly into the notch 110 defined in the anvil assembly 24 as the drive assembly 130 is advanced to prevent advancement of the drive assembly 130 to its advanced position. As such, when a knife 66 is not present in the staple cartridge 28, the stapling device 10 (FIG. 1) cannot be fired. Although the notch is shown to be defined in the anvil assembly 24, it is also envisioned that the notch 110 could be defined in the cartridge assembly 26.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. A tool assembly (16) comprising:
a cartridge assembly (26) and an anvil assembly (24) that are coupled together such that the tool assembly is movable between open and clamped positions, the tool assembly defining a notch (110), the cartridge assembly (26) including a staple cartridge (28) having a cartridge body (32), staples (38), pushers (36), and an actuation sled and knife assembly (34), the actuation sled and knife assembly (34) including an actuation sled (34a) and a knife (66), the actuation sled and knife assembly (34) movable within the cartridge body (32) from a retracted position to an advanced position to eject the staples (38) from the cartridge body (32); and
a drive assembly (130) movable between a retracted position and an advanced position and including a flexible drive beam (132), a working member (134), and a lockout mechanism (136), the flexible drive beam (132) having a proximal portion and a distal portion, the working member (134) supported on the distal portion of the flexible drive beam and including a first beam (138), a second beam (140), and a vertical strut (142) connecting the first beam to the second beam, the lockout mechanism (136) supported on the working member (134) and movable between a locked position and an unlocked position, wherein in the locked position, the lockout mechanism (136) is received within the notch (110) defined by the tool assembly to prevent movement of the drive assembly (130) to the advanced position,
wherein the lockout mechanism (136) includes a lockout member (160) that is pivotably supported on the working member (134) of the drive assembly (130),
**characterized in that**:
the knife (66) is supported on the actuation sled (34a) for movement between raised and lowered positions; and the vertical strut (142) of the working member defines a cutout (150) and the lockout member (160) is confined within the cutout (150) in the unlocked position and extends from the cutout (150) in the locked position.

2. The tool assembly of claim 1, wherein the lockout mechanism (136) further includes a biasing member (162) that is positioned to urge the lockout member (160) towards the locked position.

3. The tool assembly of claim 2, wherein the vertical strut (142) defines a cam channel (152) that extends upwardly in a proximal direction along the vertical strut, the cam channel (152) communicating with the cutout (150).

4. The tool assembly of claim 3, wherein the lockout member (160) includes a finger (172) that extends into the cam channel (152).

5. The tool assembly of claim 4, wherein the knife (66) includes a protrusion (82) that is received in the cam channel (152) when the drive assembly (130) is moved from its retracted position towards its advanced position, the protrusion (82) being movable through the cam channel (152) to move the knife (66) from the lowered position to the raised position.

6. The tool assembly of claim 5, wherein the protrusion (82) of the knife (66) is movable into engagement with the finger (172) of the lockout member (160) to move the lockout member from the locked position to the unlocked position.

7. The tool assembly of any preceding claim, wherein the notch (110) is defined in the anvil assembly (24).

8. The tool assembly of claim 7, wherein the anvil assembly (24) defines a knife slot (106) and the notch (110) is formed along the knife slot (106).

9. The tool assembly of any preceding claim, wherein the actuation sled (34a) includes a body having a central portion (52) and cam surfaces (54) positioned on opposite sides of the central portion.

10. A surgical stapling device (10) comprising:
an elongate body (14) having a proximal portion and a distal portion; and
a tool assembly (16) according to any preceding claim supported on the distal portion of the elongate body (14).

11. The surgical stapling device of claim 10, further including a handle assembly (12), the handle assembly coupled to the proximal portion of the elongate body (14).

## Patentansprüche

1. Werkzeugbaugruppe (16), umfassend:
eine Magazinbaugruppe (26) und eine Ambossbaugruppe (24), die so miteinander gekoppelt sind, dass die Werkzeugbaugruppe zwischen einer offenen und einer geklemmten Position bewegbar ist, wobei die Werkzeugbaugruppe eine Kerbe (110) definiert, wobei die Magazinbaugruppe (26) ein Klammermagazin (28) mit einem Magazinkörper (32), Klammern (38) und Schiebern (36) beinhaltet, und eine Betätigungsschlitten- und Messerbaugruppe (34), wobei die Betätigungsschlitten- und Messerbaugruppe (34) einen Betätigungsschlitten (34a) und ein Messer (66) beinhaltet, wobei die Betätigungsschlitten- und Messerbaugruppe (34) innerhalb des Magazinkörpers (32) aus einer zurückgezogenen Position in eine vorgeschobene Position bewegbar ist, um die Klammern (38) aus dem Magazinkörper (32) auszustoßen; und
eine Antriebsbaugruppe (130), die zwischen einer zurückgezogenen Position und einer vorgeschobenen Position bewegbar ist und einen flexiblen Antriebsträger (132), ein Arbeitselement (134) und einen Sperrmechanismus (136) beinhaltet, wobei der flexible Antriebsträger (132) einen proximalen Abschnitt und einen distalen Abschnitt aufweist, wobei das Arbeitselement (134) am distalen Abschnitt des flexiblen Antriebsträgers gelagert ist und einen ersten Träger (138), einen zweiten Träger (140) und eine den ersten Träger mit dem zweiten Träger verbindende vertikale Strebe (142) beinhaltet, wobei der Sperrmechanismus (136) am Arbeitselement (134) gelagert ist und zwischen einer verriegelten Position und einer entriegelten Position bewegbar ist, wobei in der verriegelten Position der Sperrmechanismus (136) in der durch die Werkzeugbaugruppe definierten Kerbe (110) aufgenommen ist, um eine Bewegung der Antriebsbaugruppe (130) in die vorgeschobene Position zu verhindern,
wobei der Sperrmechanismus (136) ein Sperrelement (160) beinhaltet, das schwenkbar am Arbeitselement (134) der Antriebsbaugruppe (130) gelagert ist,
**dadurch gekennzeichnet, dass**:
das Messer (66) am Betätigungsschlitten (34a) gelagert ist, um zwischen einer angehobenen und einer abgesenkten Position bewegt zu werden; und
die vertikale Strebe (142) des Arbeitselements einen Ausschnitt (150) definiert und das Sperrelement (160) in der entriegelten Position innerhalb des Ausschnitts (150) angeordnet ist und sich in der verriegelten Position aus dem Ausschnitt (150) heraus erstreckt.

2. Werkzeugbaugruppe nach Anspruch 1, wobei der Sperrmechanismus (136) ferner ein Vorspannelement (162) beinhaltet, das dazu positioniert ist, das Sperrelement (160) in Richtung der verriegelten Position zu drücken.

3. Werkzeugbaugruppe nach Anspruch 2, wobei die vertikale Strebe (142) einen Nockenkanal (152) definiert, der sich entlang der vertikalen Strebe in einer proximalen Richtung nach oben erstreckt, wobei der Nockenkanal (152) mit dem Ausschnitt (150) in Verbindung steht.

4. Werkzeugbaugruppe nach Anspruch 3, wobei das Sperrelement (160) einen Finger (172) beinhaltet, der sich in den Nockenkanal (152) erstreckt.

5. Werkzeugbaugruppe nach Anspruch 4, wobei das Messer (66) einen Vorsprung (82) beinhaltet, der im Nockenkanal (152) aufgenommen wird, wenn die Antriebsbaugruppe (130) aus ihrer zurückgezogenen Position in Richtung ihrer vorgeschobenen Position bewegt wird, wobei der Vorsprung (82) durch den Nockenkanal (152) bewegbar ist, um das Messer (66) aus der abgesenkten Position in die angehobene Position zu bewegen.

6. Werkzeugbaugruppe nach Anspruch 5, wobei der Vorsprung (82) des Messers (66) in Eingriff mit dem Finger (172) des Sperrelements (160) bewegbar ist, um das Sperrelement aus der verriegelten Position in die entriegelte Position zu bewegen.

7. Werkzeugbaugruppe nach einem der vorangehenden Ansprüche, wobei die Kerbe (110) in der Ambossbaugruppe (24) definiert ist.

8. Werkzeugbaugruppe nach Anspruch 7, wobei die Ambossbaugruppe (24) einen Messerschlitz (106) definiert und die Kerbe (110) entlang des Messerschlitzes (106) ausgebildet ist.

9. Werkzeugbaugruppe nach einem der vorangehenden Ansprüche, wobei der Betätigungsschlitten (34a) einen Körper mit einem mittleren Abschnitt (52) und auf gegenüberliegenden Seiten des mittleren Abschnitts positionierten Nockenflächen (54) beinhaltet.

10. Chirurgische Klammervorrichtung (10), umfassend:
einen länglichen Körper (14) mit einem proximalen Abschnitt und einem distalen Abschnitt und
eine Werkzeugbaugruppe (16) nach einem der vorangehenden Ansprüche, die am distalen Abschnitt des länglichen Körpers (14) gelagert ist.

11. Chirurgische Klammervorrichtung nach Anspruch 10, ferner beinhaltend eine an den proximalen Abschnitt des länglichen Körpers (14) gekoppelte Griffbaugruppe (12).

## Revendications

1. Ensemble outil (16) comprenant :
un ensemble cartouche (26) et un ensemble enclume (24) qui sont accouplés ensemble de sorte que l'ensemble outil soit mobile entre des positions ouverte et serrée, l'ensemble outil définissant une encoche (110), l'ensemble cartouche (26) comprenant une cartouche d'agrafes (28) ayant un corps de cartouche (32), des agrafes (38), des poussoirs (36), et un ensemble chariot d'actionnement et couteau (34), l'ensemble chariot d'actionnement et couteau (34) comprenant un chariot d'actionnement (34a) et un couteau (66), l'ensemble chariot d'actionnement et couteau (34) étant mobile à l'intérieur du corps de cartouche (32) d'une position rétractée à une position avancée pour éjecter les agrafes (38) du corps de cartouche (32) ; et
un ensemble d'entraînement (130) mobile entre une position rétractée et une position avancée et comportant une poutre d'entraînement flexible (132), un élément de travail (134), et un mécanisme de verrouillage (136), la poutre d'entraînement flexible (132) ayant une partie proximale et une partie distale, l'élément de travail (134) étant supporté sur la partie distale de la poutre d'entraînement flexible et comprenant une première poutre (138), une seconde poutre (140), et un montant vertical (142) reliant la première poutre à la deuxième poutre, le mécanisme de verrouillage (136) étant supporté sur l'élément de travail (134) et mobile entre une position verrouillée et une position déverrouillée, dans la position verrouillée, le mécanisme de verrouillage (136) étant reçu dans l'encoche (110) définie par l'ensemble outil pour empêcher le mouvement de l'ensemble d'entraînement (130) vers la position avancée,
le mécanisme de verrouillage (136) comprenant un élément de verrouillage (160) qui est supporté pivotant sur l'élément de travail (134) de l'ensemble d'entraînement (130),
**caractérisé en ce que** :
le couteau (66) est supporté sur le chariot d'actionnement (34a) pour un mouvement entre des positions relevée et abaissée ; et
l'entretoise verticale (142) de l'élément de travail définissant une découpe (150) et l'élément de verrouillage (160) étant confiné dans la découpe (150) en position déverrouillée et s'étendant à partir de la découpe (150) en position verrouillée.

2. Ensemble outil selon la revendication 1, le mécanisme de verrouillage (136) comprenant en outre un élément de sollicitation (162) qui est positionné pour pousser l'élément de verrouillage (160) vers la position verrouillée.

3. Ensemble outil selon la revendication 2, l'entretoise verticale (142) définissant un canal de came (152) qui s'étend vers le haut dans une direction proximale le long de l'entretoise verticale, le canal de came (152) communiquant avec la découpe (150).

4. Ensemble outil selon la revendication 3, l'élément de verrouillage (160) comprenant un doigt (172) qui s'étend dans le canal de came (152).

5. Ensemble outil selon la revendication 4, le couteau (66) comprenant une saillie (82) qui est reçue dans le canal de came (152) lorsque l'ensemble d'entraînement (130) est déplacé de sa position rétractée vers sa position avancée, la saillie (82) étant mobile à travers le canal de came (152) pour déplacer le couteau (66) de la position abaissée à la position relevée.

6. Ensemble outil selon la revendication 5, la saillie (82) du couteau (66) étant mobile en prise avec le doigt (172) de l'élément de verrouillage (160) pour déplacer l'élément de verrouillage de la position verrouillée à la position déverrouillée.

7. Ensemble outil selon l'une quelconque des revendications précédentes, l'encoche (110) étant définie dans l'ensemble enclume (24).

8. Ensemble outil selon la revendication 7, l'ensemble enclume (24) définissant une fente de couteau (106) et l'encoche (110) étant formée le long de la fente de couteau (106).

9. Ensemble outil selon l'une quelconque des revendications précédentes, le chariot d'actionnement (34a) comprenant un corps ayant une partie centrale (52) et des surfaces de came (54) positionnées sur des côtés opposés de la partie centrale.

10. Dispositif d'agrafage chirurgical (10) comprenant :
un corps allongé (14) ayant une partie proximale et une partie distale ; et
un ensemble outil (16) selon l'une quelconque des revendications précédentes supporté sur la partie distale du corps allongé (14).

11. Dispositif d'agrafage chirurgical selon la revendication 10, comportant en outre un ensemble poignée (12), l'ensemble poignée étant accouplé à la partie proximale du corps allongé (14).
